# EUROPÄISCHE PATENTSCHRIFT

(11) **EP 2 558 271 B1**
(45) Veröffentlichungstag und Bekanntmachung des Hinweises auf die Patenterteilung: **02.03.2016**
(21) Anmeldenummer: 11708727.0
(22) Anmeldetag: 09.03.2011
(51) Int. Cl.: A61M 37/00, B29C 47/08, B29C 47/00, B29L 31/00

(54) **KANÜLENVORRICHTUNG UND VERFAHREN UND VORRICHTUNG ZUM BESTÜCKEN EINER KANÜLENVORRICHTUNG MIT EINEM IMPLANTAT**
CANNULA DEVICE AND METHOD AND DEVICE FOR PROVIDING A CANNULA DEVICE WITH AN IMPLANT
DISPOSITIF DE CANULE ET PROCÉDÉ ET DISPOSITIF POUR ÉQUIPER UN DISPOSITIF DE CANULE D'UN IMPLANT

(30) Priorität: 13.04.2010 DE 102010014791
(43) Veröffentlichungstag der Anmeldung: 20.02.2013
(73) Patentinhaber: Acino AG, 83714 Miesbach (DE)
(72) Erfinder: SPILGIES, Heiko, 81373 München (DE); MÜLLER, Andreas, 82025 Taufkirchen (DE); SCHÖNBACH, Franz, 83730 Fischbachau (DE)
(74) Vertreter: Forstmeyer, Dietmar
(86) Internationale Anmeldenummer: PCT/EP2011/001161
(87) Internationale Veröffentlichungsnummer: WO 2011/128015

(56) Entgegenhaltungen:
- WO-A2-2006/052645
- US-A1- 2003 233 101
- US-A1- 2007 292 473

## Beschreibung

Die Erfindung betrifft eine Kanülenvorrichtung sowie eine Bestückungsvorrichtung zum Bestücken eines Kanülenhalters mit einem Implantat. Ferner betrifft die Erfindung ein Verfahren zum Bestücken der Kanülenvorrichtung mit einem Implantat.

Implantationsvorrichtungen werden beispielsweise bei der Behandlung von Tumorpatienten eingesetzt und dienen dazu, Tumorpatienten ein Medikament subkutan zu verabreichen. Dazu werden als Implantate Stäbchen aus einem biologisch abbaubaren Material, wie z.B. einem Kunststoff, hergestellt. Das Implantat gibt im Körper des Patienten einen Wirkstoff ab, der beispielsweise eine Testosteronsenkung und damit eine Hemmung des Tumorwachstums bewirkt. Der Abbau des Implantats erfolgt beispielsweise durch Hydrolyse aufgrund des natürlichen Wasserhaushalts im menschlichen Körper. Der Zeitraum der Abgabe des Medikaments im Körper des Patienten erstreckt sich typischerweise über ca. vier Wochen.

Für das Einbringen des Implantats in den menschlichen Körper wird die Implantationsvorrichtung verwendet. Diese besteht üblicher Weise aus drei Funktionsteilen: einer Einstichkanüle, die das Implantat aufweist, einem Kanülenhalter, der mit der Kanüle dauerhaft verbunden sein bzw. die Kanüle umfassen kann, und einem Applikator, der das Implantat aus der Einstichkanüle in das Gewebe des Patienten appliziert.

Gemäß dem Stand der Technik wird ein Implantat in Form eines Stäbchens mit einer Pinzette und mit Hilfe eines Kolbenstabs in die proximale Öffnung der Kanüle (die Kanülenspitze) eingeführt. Um das somit lose in der Kanüle vorliegende Implantatstäbchen gegen unbeabsichtigtes Herausfallen zu sichern, wird anschließend über die proximale Öffnung der Kanüle eine Lösung aus Kunststoff auf das Implantat in der Kanüle appliziert, um so das Implantatstäbchen zu fixieren. Diese Lösung aus Kunststoff kann mit einer Dosierpipette als Tropfen in einer vordefinierten Menge in die Kanüle eingebracht werden. Der Tropfen sitzt dann proximal vor dem Implantatsstäbchen und haftet sowohl an der Innenwand der Kanüle als auch an dem Implantatstäbchen und härtet bei Raumtemperatur aus. Er sichert somit ein Herausfallen des Implantatstäbchens aus der Kanüle.

Soll ein derartiges Implantatstäbchen appliziert werden, so wird es mit Hilfe eines am distalen Ende des Implantatstäbchens angreifenden Stabkolbens gegen den ausgehärteten Kunststofftropfen gepresst, der sich dabei löst und das Implantatstäbchen freigibt.

Dieser Stand der Technik hat den Nachteil, dass mehrere Arbeitsschritte erforderlich sind, um eine Kanüle mit Implantatstäbchen für einen Einsatz bereitzustellen. Außerdem ist es schwierig, eine reproduzierbare Position des Kunststofftropfens in der Kanüle zu gewährleisten. Schließlich wird die Oberfläche des Implantatstäbchens, über die der Wirkstoff freigesetzt werden soll, unterschiedlich stark von der Kunststofflösung abgedichtet, so dass Teile der Oberfläche zeitweise nicht für die Freisetzung des Wirkstoffs aus dem Implantatstäbchen zur Verfügung stehen, was nachteilig eine unregelmäßige Abgabe des Wirkstoffs zur Folge haben kann. Auch wird bei Applikation des Implantats der Kunststoffpfropfen mit injiziert, wodurch ein Patient zusätzlich belastet werden kann.

WO 2006/052645 A2 beschreibt ein Verfahren zum Verschließen der Spitze einer Injektionskanüle mit einem Verschlußmaterial.

US 2003/233101 A1 beschreibt eine Injektionskanüle mit verstopfter Spitze, die ein oder mehrerer Implantate enthält. US 2007/292473 A1 beschreibt ein Verfahren zum Befüllen einer Injektionskanüle mit einem festen oder halbfesten Material für die Injektion.

Eine Aufgabe der vorliegenden Erfindung ist es, eine Lösung zu finden, bei der das Implantat sicher und reproduzierbar in der Kanüle fixiert wird und gleichzeitig sichergestellt wird, dass kein zusätzliches Material injiziert werden muss und dass die Eigenschaften des Implantats nicht durch zusätzliches Material nachteilig beeinflusst werden.

Diese Aufgabe löst die Erfindung durch Bereitstellen einer verbesserten Kanülenvorrichtung mit einem Implantat bzw. für ein Implantat, einer Bestückungsvorrichtung zum Bestücken einer Kanülenvorrichtung mit einem Implantat und eines Verfahrens zum Bestücken einer Kanülenvorrichtung mit einem Implantat. Erfindungsgemäß wird eine Kanülenvorrichtung nach Anspruch 1, ein System nach Anspruch 9 und ein Verfahren nach Anspruch 11 bereitgestellt. Vorteilhafte Weiterbildungen der Erfindung sind in den abhängigen Ansprüchen definiert.

Eine erfindungsgemäße Kanülenvorrichtung mit einem proximalen Ende und einem distalem Ende, das zur Verbindung mit einem Applikator vorgesehen ist, umfasst einen Implantatkanal, der sich von dem distalen Ende zu dem proximalen Ende der Kanülenvorrichtung erstreckt, und ein Implantat in dem Implantatkanal, wobei das Implantat durch Verfestigen eines in fließfähigem Zustand in den Implantatkanal eingebrachten Materials in dem Implantatkanal gebildet ist. Ein Vorteil dieser Kanülenvorrichtung besteht darin, dass die Herstellung des Implantats und die Bestückung der Kanülenvorrichtung mit dem Implantat in einem einzigen Verfahren und damit mit besonders wenigen Verfahrensschritten erfolgen. Dadurch werden das Fehlerrisiko und die Herstellungskosten reduziert. Erfindungsgemäß umfasst die Kanülenvorrichtung eine schlauch- oder rohrförmige Auskleidung von zumindest einem Abschnitt des Implantatkanals.

Erfindungsgemäß wird das Implantat durch Verfestigen eines fließfähigen Material gebildet. Bevorzugt umfasst das fließfähige Material alle Bestandteile des herzustellenden Implantats. Bevorzugt ist das fließfähige Material eine Polymerschmelze, die alle Bestandteile des herzustellenden Implantats umfasst. Das Implantat und damit das fließfähige Material umfasst vorzugsweise einen oder mehrere Wirkstoffe sowie eine Matrix, die aus bioabbaubaren Materialien gebildet wird. Bevorzugt werden als Matrix-bildendes Material Polymere, insbesondere Polylactid-coglyceride und/oder Polylactide und/oder Lipide eingesetzt. Besonders bevorzugt sind Poly-(lactid-co-glycolide) mit einer inhärenten Viskosität von 0,1 bis 0,7 dl/g. Besonders bevorzugt sind Poly-lactide mit einer inhärenten Viskosität von 0,1 bis 0,7 dl/g. Besonders bevorzugt sind Triglyceride, Mono- und Di-glyceride (aus Glycerin und Fettsäuren), wobei Fettsäuren langkettige Säuren mit 8 und mehr Kohlenstoffatomen sind, vorzugsweise mit 8 bis 30 Kohlenstoffatomen. Bevorzugt sind die Fettsäuren Monocarbonsäuren mit 8 bis 30 Kohlenstoffatomen, die gesättigt oder einfach oder mehrfach ungesättigt sind, und optional verzweigt sein können. Die Fettsäuren können auch partiell durch Essigsäuren ersetzt sein (acylierte Glyceride). Weiterhin können modifizierte Glyceride eingesetzt werden, wie z.B. PEGylierte Glyceride. Weiterhin kommen auch Phospholipide (z.B. Lecithin) in Frage. Die wirkstoffhaltige Matrix schmilzt oberhalb der druckabhängigen Glasübergangstemperatur. Bevorzugt wird die Masse bei Temperaturen von 50 bis 120°C, vorzugsweise von 70 bis 90°C, unter Drücken von bis zu 20 kN, vorzugsweise von 0,5 bis 2 kN, fließfähig. Das Schmelzen der Masse sowie deren Glasübergangstemperatur wird bevorzugt mittels DSC (Dynamische Differenzkalorimetrie) gemessen: Bei Phasenänderungen wie Schmelzen oder Verdampfen kommt es zu Temperaturänderungen delta-T im Vergleich zu einer leeren Probe T_{Ref}. Wird die Probe im Messgerät erhitzt, fließt Wärme durch Probe und Referenz. Verändert sich eine Probe während der Messung, z.B. durch Schmelzen, entsteht eine Differenz im Wärmestrom von Probe und Referenz, die durch Integration der delta-T-T_{Ref}-Kurve aufgezeichnet wird. Das DSC-Verfahren sowie geeignete Messgeräte sind im Fachgebiet bekannt.

Bevorzugt wird das fließfähige Material unter diesen Bedingungen in den Implantatkanal eingebracht, beispielsweise durch Extrusion. Besonders bevorzugt wird das fließfähige Material bei einer Temperatur im Bereich von 50 bis 120°C bei einem Druck von bis zu 20 kN in die Schlauchmatrix extrudiert.

Als Wirkstoffe können erfindungsgemäß z.B. LHRH-Analoga eingesetzt werden, insbesondere Goserelin und/oder Leuprorelin.

In einer anderen Ausführungsform umfasst eine Kanülenvorrichtung mit einem proximalen Ende und einem distalen Ende, das zur Verbindung mit einem Applikator vorgesehen ist, einen Implantatkanal, der sich von dem distalen Ende zu dem proximalen Ende der Kanülenvorrichtung erstreckt, und eine rohrförmige Auskleidung zumindest eines Teils des Implantatkanals. Die Auskleidung mit einem Material, das sich von dem sonst für die Kanülenvorrichtung verwendeten Material unterscheiden kann, ermöglicht eine Anpassung der Materialeigenschaften an das Implantatmaterial und andere Randbedingungen. Optional umfasst die Kanülenvorrichtung ferner einen Kolbenstab, der in dem Implantatkanal angeordnet ist, und der vorzugsweise einen Dichtkolben aufweist, der den Implantatkanal am Ort des Dichtkolbens abschließt. Dieser Kolbenstab ermöglicht, wie unten beschrieben wird, eine besonders einfache und gleichzeitig genaue Dimensionierung des Implantats.

Die Auskleidung des Implantatkanals wird vorzugsweise durch ein Rohr oder einen Schlauch gebildet, das bzw. der in eine Bohrung in der Kanülenvorrichtung eingesetzt ist. Vorzugsweise weist der Implantatkanal im Bereich der Bohrung einen vergrößerten Innendurchmesser auf. Vorzugsweise umfasst das Rohr bzw. der Schlauch ein Kunststoffpolymer oder -copolymer. Bevorzugte Polymere sind inert gegenüber einer Reaktion mit der Extrusionsmasse. Bevorzugte Kunststoffpolymere sind Polyfluorpolymere, wie beispielsweise PFA, PTFA oder FEP, insbesondere Polytetrafluorethylen (z.B. Teflon). Diese können entweder einzeln oder zusammen mit anderen Polymeren, auch copolymerisiert, verwendet werden.

Das Material der Auskleidung sollte flexibel sein, um eine Entnahme des Implantats nach Erstarren zu ermöglichen, da sich die eingebrachte Masse nach Entfernung des Extrusionsdruckes leicht ausdehnen kann. Dabei ist auf eine ausreichende Stabilität der Injektionseinheit zu achten, um eine gute Handhabung durch den Anwender (Arzt) zu ermöglichen. Der Ausgleich zwischen den erforderlichen elastischen Eigenschaften der Rohrauskleidung und den mechanischen Eigenschaften der Kanülenvorrichtung kann durch geeignete Wahl der Materialien für Vorrichtung und Auskleidung erreicht werden.

Besonders bevorzugt ist die Kombination einer Kanülenvorrichtung aus Polycarbonat mit einer Implantatkanal-Auskleidung aus Teflon. Es wurde gefunden, dass die Kombination von Polycarbonat und Teflon besonders stabil gegenüber einer Belastung durch Gamma-Strahlung und Hitze ist, wie sie z.B. bei der Sterilisation auf die Kanülenvorrichtung einwirken. Die Belastbarkeit durch Gamma-Strahlung und Hitze wurde empirisch bestimmt. Dabei ergab sich die Kombination von Polycarbonat mit eingelegtem Teflonschlauch als besonders stabil.

Der Kolbenstab (Stempel) muss dem zu erwartenden Extrusionsdruck von bis zu 20 kN standhalten können und gewährleisten, dass sich das Implantat nach Erstarren der extrusionsmasse leicht vom Stempel lösen kann. Bevorzugt besteht der Kolbenstab (Stempel) aus Edelstahl, dessen Klebeigenschaften vorzugsweise durch eine geeignete Oberflächenbehandlung, beispielsweise mit Silikon, verringert werden können.

Zum Einsetzen der Auskleidung weist die Bohrung einen Innendurchmesser und das Rohr einen Außendurchmesser auf, die größer sind als der Innendurchmesser des Implantatkanals. Das Rohr kann starr oder flexibel bzw. schlauchartig sein. Die Bohrung und das Rohr können jeweils eine geringere Länge aufweisen als der Implantatkanal, so dass nur ein Abschnitt des Implantatkanals durch das Rohr ausgekleidet ist, insbesondere der Abschnitt, der von dem Implantat ausgefüllt wird bzw. in den das Implantat eingebracht wird. Dieser Abschnitt grenzt vorzugsweise an das distale Ende der Kanülenvorrichtung an. Vorzugsweise ist der Innendurchmesser eines sich an die Bohrung anschließenden Abschnitts des Implantatkanals kleiner als der Innendurchmesser der Bohrung und der Außendurchmesser des Rohrs. Der dazwischen liegende Absatz verhindert während des Implantierens ein Verrutschen des Rohres.

Der Innendurchmesser der schlauch- oder rohrförmigen Auskleidung hängt von dem gewünschten Implantatdurchmesser ab. Dieser beträgt üblicherweise 0,5 bis 5 mm, und bevorzugt 1 bis 2 mm. Bevorzugt entspricht der Innendurchmesser der Auskleidung dem gewünschten Implantatdurchmesser. Bevorzugt beträgt der Durchmesser von 1 bis 2 mm, weiter bevorzugt von 1,2 bis 1,8 mm, und besonders bevorzugt etwa 1,5 mm. Die Untergrenze wird durch die mechanische Stabilität des Implantats bestimmt. Implantate mit einem Durchmesser von weniger als 0,5 mm sind in der Regel nicht stabil genug. Die Obergrenze wird durch die Akzeptanz der Injektionsnadel bedingt, deren Innendurchmesser größer als der Implantatsdurchmesser sein muss. Bevorzugt ist der Innendurchmesser der Injektionsnadel 0,1 bis 0,5 mm größer als der Implantatsdurchmesser. Injektionsnadeln mit einem Innendurchmesser von mehr als 5,5 mm verursachen große Wunden und sind nicht akzeptabel. Besonders bevorzugt beträgt der Bohrungsdurchmesser von 2 bis 3 mm bei gleichzeitigem Durchmesser des Implantatkanals von 1 bis 2 mm.

Bevorzugt ist die Länge der Auskleidung gleich lang oder länger als die gewünschte Implantatlänge. Bevorzugte Implantatlängen betragen von 10 bis 25 mm. Die Implantatlänge wird abhängig von Faktoren eingestellt werden, wie beispielsweise der gewünschten Anwendung oder Dosierung, der Implantatzusammensetzung, der Wirkstoffkonzentration etc. Entsprechend beträgt die Auskleidungslänge bevorzugt von 10 bis 30 mm, weiter bevorzugt von 20 bis 30 mm, und besonders bevorzugt von 25 bis 30 mm. Damit kann die erfindungsgemäße Kanülenvorrichtung vorteilhaft einen universellen Einsatz bei verschiedenen Produkten ermöglichen.

Die beschriebenen Kanülenvorrichtungen können vielfältig variiert und für verschiedene Anwendungen und Rahmenbedingungen optimiert werden. So kann beispielsweise die Länge des Implantats kleiner als die Länge des Implantatkanals sein. Ferner kann der Implantatkanal im Bereich des Implantats einen geringeren Innendurchmesser aufweisen als an anderen Stellen. Durch diese unterschiedlichen Innendurchmesser wird ein sicheres Halten des Implantats an seinem vorgesehenen Ort während der Lagerung und des Transports und gleichzeitig ein leichtes Verschieben bei der Implantation ermöglicht.

Die Kanülenvorrichtung ist vorzugsweise ein Kanülenhalter und kann eine Kanüle umfassen, die mit dem Kanülenhalter kraft-, form- oder stoffschlüssig verbunden ist. Alternativ ist die Kanülenvorrichtung für eine Verbindung mit einer Kanüle, beispielsweise über eine Luer- oder Luer-Lock-Verbindung, ausgebildet. Der Implantatkanal weist vorzugsweise zumindest im Bereich des Implantats bzw. einer für die Aufnahme eines Implantats vorgesehenen Implantatkammer einen geringeren Innendurchmesser auf als die Kanüle. Dies hat die bereits erwähnten Vorteile. Die Implantatkammer, in der das Implantat gebildet wird oder angeordnet ist, kann aber auch in der Kanüle liegen, wodurch eine besonders einfache Bauform erzielt wird, die geringe Herstellungskosten bewirkt.

Als Kanüle kann eine übliche Kanüle mit den gewünschten Abmessungen verwendet werden.

Die Kanülenvorrichtung umfasst vorzugsweise ein transparentes Material und/oder ein Sichtfenster aus transparentem Material. Das Sichtfenster ist vorzugsweise nahe dem proximalen Ende der Kanülenvorrichtung bzw. nahe der Kanüle angeordnet ist. Es erlaubt vorzugsweise eine Beobachtung des Implantatkanals mit einer Blickrichtung senkrecht zum Implantatkanal. Dadurch kann vor dem Implantieren optisch geprüft werden, ob die Kanülenvorrichtung mit einem Implantat bestückt ist, und/oder beim Implantieren die Bewegung des Implantats beobachtet werden. Das Fenster kann auch eine Aussparung in der Kanüle sein oder durch die Transparenz der Kanülenbefestigung im Bereich zwischen rohr- bzw. schlauchförmiger Auskleidung des Implantatkanals ausgebildet werden. Diese Anordung bietet den Vorteil, dass das Implantat nach Verfestigung im Rohr bzw. Schlauch etwas verschoben werden kann, um dann im Fenster sichtbar zu werden. Sowohl das Material der Kanülenvorrichtung als auch das transparente Material des Sichtfensters und das Material des Dichtkolbens sind vorzugsweise jeweils pharmazeutisch inert. Bevorzugte Materialien sind Kunststoffe, insbesondere Silikon, Polycarbonat, wie z.B. von der Fa. Bayer unter dem Handelsnamen Makrolon verkauft, Polypropylen, Polyethylen und Polytetrafluorethylen. Falls ein Sichtfenster im Bereich des Nadelhalters gewünscht ist, wird Polycarbonat als Material dafür bevorzugt. Für ein Sichtfenster im Bereich der Injektionskanüle wird eine Aussparung in der Kanüle ausgebildet. Nicht-transparente Bereiche können, falls gewünscht, durch Einbringen geeigneter undurchsichtiger Füllstoffe erhalten werden. Für den bei der Produktion benötigten Dichtkolben wird bevorzugt eine Beschichtung aus Silikon oder Polytetrafluorethylen (Teflon) auf einen Kolben aus optional gehärteten Edelstahl aufgebracht. Die Materialien für Kanülenvorrichtung und Einsatz müssen temperaturbeständig bis zu der Temperatur sein, mit der das Implantat extrudiert wird (50 bis 120°C). Weiterhin dürfen sie bei Bestrahlung mit Gammastrahlen zur Sterilisation keine Trübung erfahren. Es wurde gefunden, dass diese Erfordernisse von kommerziell erhältlichen Materialien erfüllt werden, die hinsichtlich ihrer Monomer- und/oder Additivzusammensetzung den gesetzlichen Vorgaben für zulässige Kunststoffe genügen, wie z.B. der EU-Richtlinie 2002/72/EG über "Materialien und Gegenstände aus Kunststoff, die dazu bestimmt sind, mit Lebensmitteln in Berührung zu kommen", der deutschen "Bedarfsgüterverordnung", der amerikanischen FDA-modifizierten ISO 10993-1 "Biological Evaluation of Medical Devices", der Europäischen Pharmacopoe (4. Ergänzung 2002, 3.2.2), der US Pharmacopeia XXII Class VI (Biokompatibilität), o.ä. Ein bevorzugtes Material ist ein Polycarbonat, das unter der Bezeichnung "Makrolon 2858" von der Fa. Bayer, z.B. farblos (Makrolon 2858 550115), erhältlich ist, und den gesetzlichen Vorgaben gemäß Materialspezifikation und Unbedenklichkeitsbescheinigung genügt.

Das Implantat weist vorzugsweise einen Kunststoff auf, der einen pharmazeutischen Wirkstoff enthält bzw. mit diesem gemischt ist. Besonders bevorzugt umfasst das Implantat einen biologisch abbaubaren Kunststoff. Besonders bevorzugt sind biologisch abbaubare Kunststoffe, die aufgrund des natürlichen Wasserhaushalts im menschlichen Körper durch Hydrolyse abgebaut werden, nachdem das Implantat einem Patienten vorzugsweise subkutan injiziert wurde. Bevorzugte Beispiele für biologisch abbaubare Kunststoffe umfassen Polymere und Copolymere aus Milchsäure und/oder Glycolsäure, insbesondere Polylactide und Poly-lactid-co-glycolide. Weiter bevorzugt sind Polyester und Lipide. Besonders bevorzugte Implantate umfassen eine Kunststoff-Wirkstoff-Kombination aus Poly-lactid-co-glycolid und Goserelin.

Das proximale Ende der Kanülenvorrichtung weist vorzugsweise eine Kanülenkupplung zum kraft-, stoff- oder formschlüssigen Verbinden des proximalen Endes der Kanülenvorrichtung mit der Kanüle auf. Das distale Ende der Kanülenvorrichtung weist vorzugsweise eine Kanülenkupplung zum kraft-, stoff- oder formschlüssigen Verbinden des distalen Endes der Kanülenvorrichtung mit einem Applikator auf.

Besonders bevorzugt wird am distalen Ende der Kanülenvorrichtung eine Modifikation einer üblichen Luer-Lock-Kupplung verwendet, bei der das am Applikator angebrachte Gegenstück des Luer-Locks im Gegensatz zu einem üblichen Luer-Lock keinen Innenkegel besitzt. Ein Eindringen des Kegels in das distale Ende des Kanülenhalters ist bei der Vorrichtung der vorliegenden Erfindung nicht möglich, da bei der Extrusion die Extruderdüse dicht mit dem Kanülenhalter abschließen können muss.

Eine Bestückungsvorrichtung zum Bestücken eines Implantatkanals einer Kanülenvorrichtung mit einem Implantat umfasst eine Extrusionsdüse zum extrudieren eines fließfähigen Implantatmaterials und eine Dichtfläche an der Extrusionsdüse. Eine Haltevorrichtung hält eine Kanülenvorrichtung an der Extrusionsdüse derart, dass die Kanülenvorrichtung an der Dichtfläche anliegt. Eine Materialzuführeinrichtung ist zum Zuführen des fließfähigen Implantatmaterials durch die Extrusionsdüse in den Implantatkanal vorgesehen, wobei das Implantatmaterial vorzugsweise frei von Luftblasen zugeführt wird. Die Bestückungsvorrichtung umfasst vorzugsweise ferner einen Anschlag für einen Kolbenstab, der in einem Implantatkanal einer von der Halteeinrichtung an der Extrusionsdüse gehaltenen Kanülenvorrichtung angeordnet und entlang des Implantatkanals bewegbar ist. Durch den Anschlag wird auf besonders einfache und zuverlässige Weise die Länge und damit die Masse des Implantats festgelegt.

Die Vorrichtung für Vorschub und Positionierung besteht vorzugsweise aus optional gehärteten Edelstahl, wobei die mit der Extrusionsmasse in Berührung kommenden Oberflächen optional mit einer inerten Beschichtung aus Silikon oder Polytetrafluorethylen (Teflon) überzogen werden können, um ein Verkleben mit der Extrusionsmasse und/oder eine unerwünschte Wechselwirkung mit eventuell aggresiven oder korrodierenden Bestandteilen derselben zu verhindern. Als Kolbenstab kann ein wie oben beschriebener Dichtkolben verwendet werden. Bevorzugt besteht dieser aus optional gehärteten Edelstahl, ebenfalls mit einer optionalen Beschichtung aus Silikon oder Polytetrafluorethylen (Teflon).

Zur Extrusion kann jeder übliche Extruder verwendet werden, wie beispielsweise ein 1-Schnecken-extruder, ein 2-SchneckenExtruder, ein Kolbenextruder oder dergleichen.

Ein Verfahren zum Bestücken einer Kanülenvorrichtung umfasst folgende Schritte: Halten der Kanülenvorrichtung an einer Extrusionsdüse so, dass die Kanülenvorrichtung an einer Dichtfläche der Extrusionsdüse anliegt; Zuführen eines fließfähigen Implantatmaterials durch die Extrusionsdüse in einen Implantatkanal der Kanülenvorrichtung; Verfestigen des Implantatmaterials in dem Implantatkanal. Das Verfestigen erfolgt, insbesondere im Falle eines thermoplastischen Implantatmaterials, vorzugsweise durch Abkühlen oder durch Polymerisation. Die Kanülenvorrichtung kann vor oder nach dem Verfestigen von der Extrusionsdüse getrennt werden. Dieses Verfahren integriert die beiden Schritte des Herstellens des Implantats und des Bestückens der Kanülenvorrichtung mit dem Implantat. Dadurch wird das Verfahren besonders einfach, schnell und zuverlässig. Insbesondere entfallen das Bereithalten und Säubern einer Form für das Implantat, die Handhabung des Implantats nach seiner Herstellung und das Einführen des Implantats in die Kanülenvorrichtung. Die Gefahr eines Verlusts, einer Beschädigung oder Verschmutzung des Implantats wird dadurch minimiert.

Die Parameter der einzelnen Herstellungsschritte werden in Abhängigkeit von der Extrusionsmasse bestimmt werden. Übliche Verfahrensschritte bei der Herstellung von Implantaten umfassen eine kalte Vorkompression bei 1000 bis 5000 N für 10 bis 30 Minuten, gefolgt von einer warmen Vorkompression bei 70 bis 120°C und 200 bis 1000 N für 10 bis 60 Minuten und der Extrusion bei 70 bis 120°C und 100 bis 2000 N. Weitere-Bedirigungen der einzelnen Verfahrensschritte können beispielsweise WO2007/107328 A1 entnommen werden (S. 10-11).

Vorzugsweise wird vor dem Zuführen des fließfähigen Implantatmaterials ein Kolbenstab in den Implantatkanal eingeführt. Dieser wird beim Zuführen des Implantatmaterials aus dem Implantatkanal verdrängt. Der Kolbenstab weist vorzugsweise einen Dichtkolben auf, der den Implantatkanal an seinem Ort verschließt. Das Implantatmaterial wird vorzugsweise so lange zugeführt, bis der Kolbenstab an einen Anschlag anschlägt. Dieser Kolbenstab erlaubt eine besonders einfache und zuverlässige Bestimmung der Länge und damit der Masse des Implantats.

Erfindungsgemäß wird ferner vor dem Zuführen des fließfähigen Implantatmaterials und insbesondere auch vor dem Halten bzw. Verbinden der Kanülenvorrichtung mit der Extrusionsdüse ein Rohr in eine Bohrung in der Kanülenvorrichtung eingesetzt. Um ein Verkleben des Implantats mit der Kanülenvorrichtung bzw. dem Schlauch zu verhindern, wird das Implantat vorzugsweise nach dem verfestigen oder nach einer Teilverfestigung des Implantatmaterials verschoben, ohne dabei die Kanülenvorrichtung zu verlassen.

Nachfolgend werden bevorzugte Ausführungsbeispiele der vorliegenden Erfindung wird anhand der beigefügten Figuren näher erläutert.
- Figur 1: zeigt mit den schematischen Ansichten A) und B) Querschnitte durch eine Implantationsvorrichtung gemäß einer ersten Ausführungsform der Erfindung;
- Figur 2: zeigt mit den schematischen Ansichten A) und B) Querschnitte durch eine Implantationsvorrichtung gemäß einer zweiten Ausführungsform der Erfindung;
- Figur 3: zeigt mit den schematischen Ansichten A), B) und C) das Bestücken eines Kanülenhalters mit einem Implantat;
- Figur 4: zeigt mit den schematischen Ansichten A), B) und C) das Bestücken einer Implantationsvorrichtung mit einem Implantat;
- Figur 5: zeigt mit den schematischen Ansichten A), B) und C) eine Implantationsvorrichtung beim Applizieren des Implantats;
- Figur 6: zeigt eine Prinzipskizze eines neuen Applikators;
- Figur 7: zeigt eine teilweise perspektivische Prinzipskizze einer Bestückungsvorrichtung.

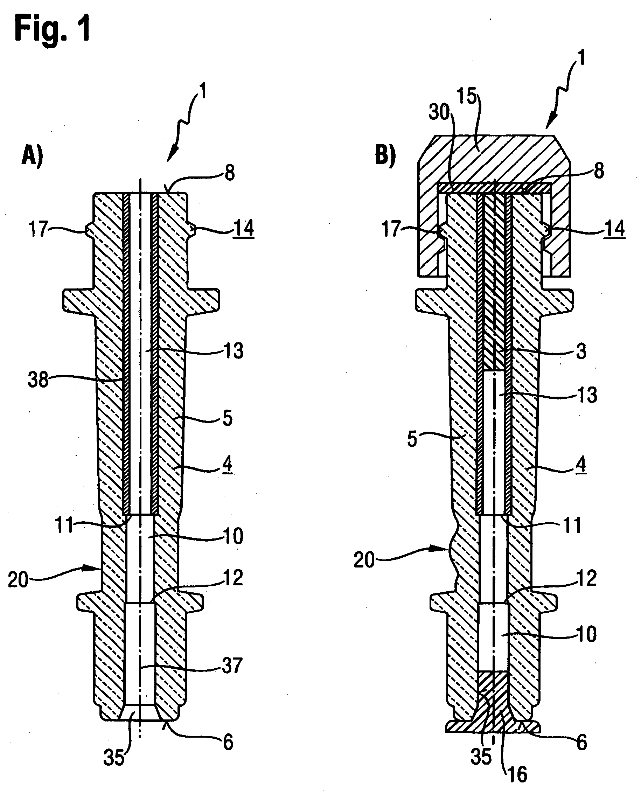

Figur 1 zeigt mit den schematischen Ansichten A) und B) Querschnitte durch eine Implantationsvorrichtung 1 gemäß einer ersten Ausführungsform der Erfindung. Dazu zeigt Figur 1 A) eine Kanülenvorrichtung 4, insbesondere einen Kanülenhalter, der eine Implantationsvorrichtung 1 nebst einem Gehäuse 5 mit einem proximalen Ende 6 und einem distalen Ende 8 aufweist. Entlang einer Kanülenhalterachse 37 ist ein zentraler Kanal angeordnet, der einen Implantatkanal 10 bildet und einen ersten Absatz 11 und einen zweiten Absatz 12 aufweist, wobei der Kanalquerschnitt zwischen den Absätzen 11 und 12 vermindert ist. Der Absatz 12 dient als Anschlag für eine Kanüle in Form einer Injektionsnadel, die über eine proximale Öffnung 35 des Kanülenhalters 4 bis zum Absatz 12 eingeführt werden kann und dort mit der Kanülenvorrichtung 4 vorzugsweise stoff-, kraft- oder formschlüssig verbunden werden kann.

Der Absatz 11 begrenzt den Bereich einer Implantatkammer 13, die in dieser Ausführungsform der Erfindung von einem Teflonrohr- bzw. -schlauchstück 38 gebildet wird und ihrerseits einen Abschnitt des Implantatkanals bildet. Dieses Teflonrohr- bzw. -schlauchstück ist in einer Bohrung der Kanülenvorrichtung 4 angeordnet. In diese Implantatkammer 13 kann direkt ein Implantatmaterial, vorzugsweise eine Wirkstoff-Polymer-Mischung, extrudiert werden. Das Gewicht eines derartigen Implantats in der Implantatkammer 13, und damit auch der Wirkstoffgehalt bei einer festgelegten Wirkstoffkonzentration in der Mischung, wird durch den Innendurchmesser des Schlauchstücks 38 und durch die Befüllungslänge des Schlauchstücks 38 mit einem Implantatmaterial bestimmt. Je nach Ausführungsform der Implantationsvorrichtung kann der Schlauchdurchmesser für die Implantatkammer 13 variiert werden.

Die Länge des Implantats wird durch einen Kolbenstab mit einem Dichtkolben festgelegt. Der Kolbenstab wird von einem Ende aus, vorzugsweise von der proximalen Öffnung 35 aus, mit dem Dichtkolben voraus in den Implantatkanal eingeführt bis der Dichtkolben mit dem anderen Ende des Implantatkanals, vorzugsweise der distalen Öffnung abschließt. Beim Befüllen der Implantatkammer mit dem Implantatmaterial wird der Dichtkolben und damit der gesamte Kolbenstab aus dem Implantatkanal verdrängt bzw. herausgeschoben, bis der Kolbenstab an einen Anschlag anschlägt. Somit wird die Länge des Implantats durch die Anordnung des Anschlags festgelegt.

Das Schlauchstück 38, und damit die Implantatkammer, erstrecken sich von dem distalen Ende 8 des Kanülenhalters 4 bis zu dem ersten Absatz 11, bei dem der Querschnitt des Implantatkanals 10 verengt ist. In diesem Übergangsbereich von der Implantatkammer 13 zu der in der proximalen Öffnung 35 angeordneten Kanüle ist Sichtfenster 20 vorgesehen, das in einer Blickrichtung quer zum Implantatkanal einen Blick in diesen ermöglicht. Dieses Sichtfenster 20 ist in der Ausführungsform der Figur 1 A) lediglich eine glatte oder polierte Außenfläche des Gehäuses 5, das in dieser Ausführungsform aus einem transparenten Kunststoff hergestellt ist.

Anstelle eines Einschiebens einer Kanüle in Form einer Injektionsnadel in die proximale Öffnung 35 kann auch auf dem Außenumfang des Kanülenhalters 4 eine zusätzliche Koppeleinrichtung vorgesehen werden, um eine Kanüle lösbar mit dem proximalen Ende des Kanülenhalters 4 zu verbinden.

Auf dem Außenmantel des Kanülenhalters 4 im Bereich des distalen Endes 8 ist eine Koppeleinrichtung 14 in Form eines Luer-Lock-Außengewindes 17 vorgesehen, das einerseits dazu dient, eine Verschlusskappe nach Befüllen der Implantatkammer 13 mit einem Implantat mediendicht aufzusetzen, und andererseits für ein Applizieren des Implantats einen Applikator an das distale Ende 8 des Kanülenhalters anzubringen.

Mit Figur 1 B) wird eine schematische Ansicht eines Querschnitts des in Figur 1 A) gezeigten Kanülenhalters 4 mit einem in die Implantatkammer 13 eingebrachten Implantat 3 gezeigt, wobei auf das distale Ende 8 des Kanülenhalters 4 eine Verschlusskappe 15 mit einem Luer-Lock-Innengewinde aufgeschraubt ist, so dass eine Dichtungsscheibe 39 in der Verschlusskappe 15 das distale Ende 8 mediendicht verschließt. Das proximale Ende 6 des Kanülenhalters 4 kann mit Hilfe eines angepassten Stopfens 16 mediendicht verschlossen werden. Im Bereich des Sichtfensters 20 ist an das transparente Gehäuse 5 eine konkave Kontur angeschliffen, um eine Linsenwirkung für die Beobachtung beim Applizieren des Implantats zu ermöglichen.

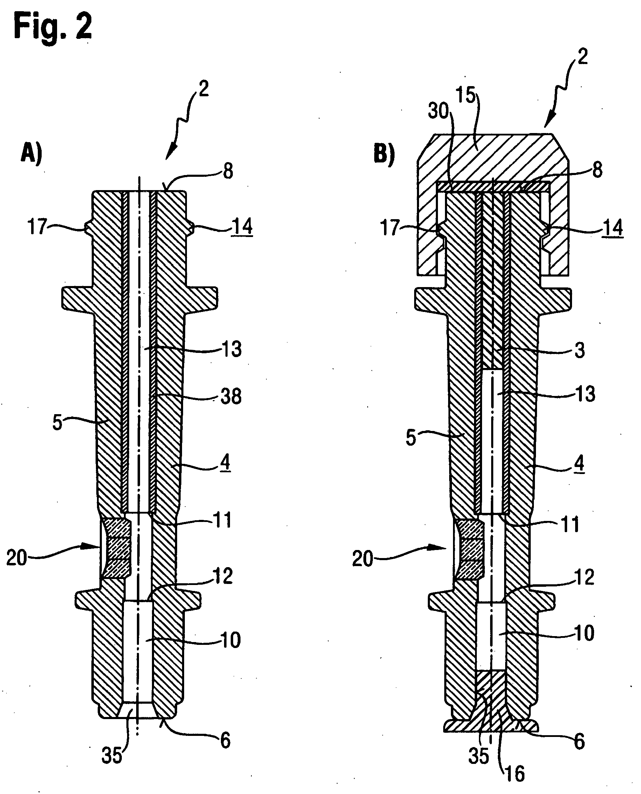

Figur 2 zeigt mit den schematischen Ansichten A) und B) Querschnitte durch eine Implantationsvorrichtung 2 gemäß einer zweiten Ausführungsform der Erfindung. Komponenten der Figur 2 mit gleichen Funktionen wie in Figur 1 werden mit gleichen Bezugszeichen gekennzeichnet und nicht extra erörtert. Der Unterschied zur ersten Ausführungsform der Erfindung besteht darin, dass das Gehäuse 5 des Kanülenhalters 4 gemäß Figur 2A) aus einem optisch nicht transparenten Material hergestellt ist. In diesem Fall ist im Bereich des Sichtfensters 20 in dem optisch nicht transparenten Material eine Öffnung ausgearbeitet, die mit einem transparenten Kunststoffmaterial aufgefüllt ist. Dadurch wird gewährleistet, dass auch hier eine Beobachtung der Bewegung des Implantats beim Applizieren im Übergangsbereich zwischen den beiden Absätzen 11 und 12 gewährleistet ist.

Figur 2 B) zeigt diese zweite Ausführungsform der Erfindung mit einem Implantat 3, das in der Implantatkammer 13 angeordnet ist, wobei während der Lagerung und des Transports eine Verschlusskappe 15, wie sie bereits aus Figur 1B) bekannt ist, und ein Stopfen 16 den Kanülenhalter 4 mediendicht abschließen.

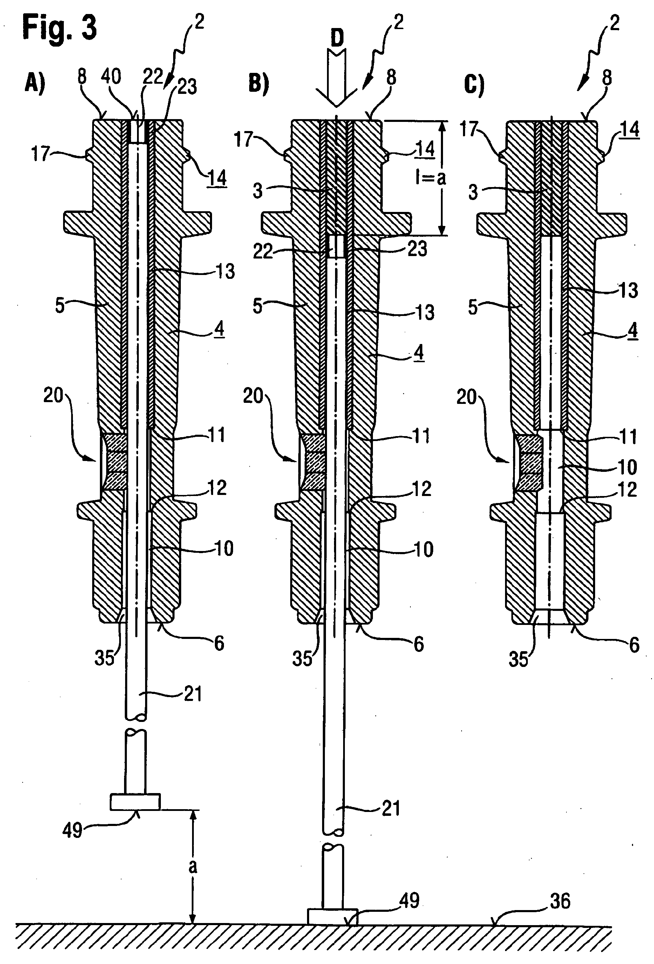

Figur 3 zeigt mit den schematischen Ansichten A), B) und C) das Bestücken eines Kanülenhalters 4 mit einem Implantat 3. Dazu wird, wie es Figur 3 A) zeigt, von der proximalen Öffnung 35 des Kanülenhalters 4 aus ein Kolbenstab 21 in den Implantatkanal 10 des Kanülenhalters eingeführt. Dazu weist der Kolbenstab 21 an seinem distalen Ende 40 einen Dichtkolben 22 auf, der mit einem medizinisch inerten Material beschichtet sein kann. Diese Beschichtung 23 sorgt dafür, dass das einzubringende Implantatmaterial nicht kontaminiert wird. Das Material der Beschichtung 23 ist vorzugsweise ebenfalls Teflon. Der Kolbenstab 21 wird durch den Implantatkanal 10 und insbesondere durch die Implantatkammer 13 so weit geschoben, bis das distale Ende 40 des Kolbenstabs 21, und damit der Dichtkolben 22, mit dem distalen Ende 8 des Kanülenhalters 4 abschließt. Das distale Ende 8 des Kanülenhalters weist eine vorzugsweise ebene Dichtfläche auf, so dass das distale Ende 8 an eine Extrusionsdüse mediendicht angesetzt werden kann. Außerdem ist ein Anschlag 36 vorgesehen, der auf einen Abstand a von dem proximalen Ende 49 des Kolbenstabs 21 eingestellt werden kann, um die Länge 1 eines Implantats 3 in der Implantatkammer 13 vorzugeben.

In Figur 3 B) befindet sich der Kanülenhalter 4 mit dem Kolbenstab 21 an einer nicht gezeigten Extrusionsdüse, die mediendicht an das distale Ende 8 des Kanülenhalters 4 angesetzt wird. In Pfeilrichtung D wird ein fließfähiges Implantatmaterial, das vorzugsweise eine Mischung aus einem Wirkstoff und einem Polymer umfasst, direkt in die Implantatkammer 13 extrudiert. Der Querschnitt der Implantatkammer 13 ist kleiner als der Innenquerschnitt des Übergangsbereichs zwischen den Absätzen 11 und 12 und auch kleiner als der Innenquerschnitt der anzusetzenden Kanüle. Beim Einbringen der Wirkstoff-Polymer-Mischung in einer Bestückungsposition eines Bestückungsautomaten wird der Kolbenstab 21 aus der proximalen Öffnung 35 des Kolbenhalters 4 verdrängt oder herausgedrückt bis der Anschlag 36 in der Bestückungsposition durch das proximale Ende 49 des Kolbenstabs 21 erreicht ist. Somit wird durch den Abstand a die Länge 1 des Implantats in der Implantatkammer 13 vorgegeben.

Figur 3 C zeigt den Kanülenhalter 4 mit einem Implantat 3 im Bereich des distalen Endes 8 der Implantatkammer 13. Dieser Kanülenhalter 4 kann anschließend mittels Bestrahlung vorzugsweise durch Gammastrahlen sterilisiert werden und steril verpackt und ausgeliefert werden. Dazu kann, wie in Figur 1 B) und Figur 2 B) gezeigt, eine Verschlusskappe mediendicht auf das distale Ende 8 und ein Stopfen auf das proximale Ende 6 des Kanülenhalters 4 aufgebracht werden.

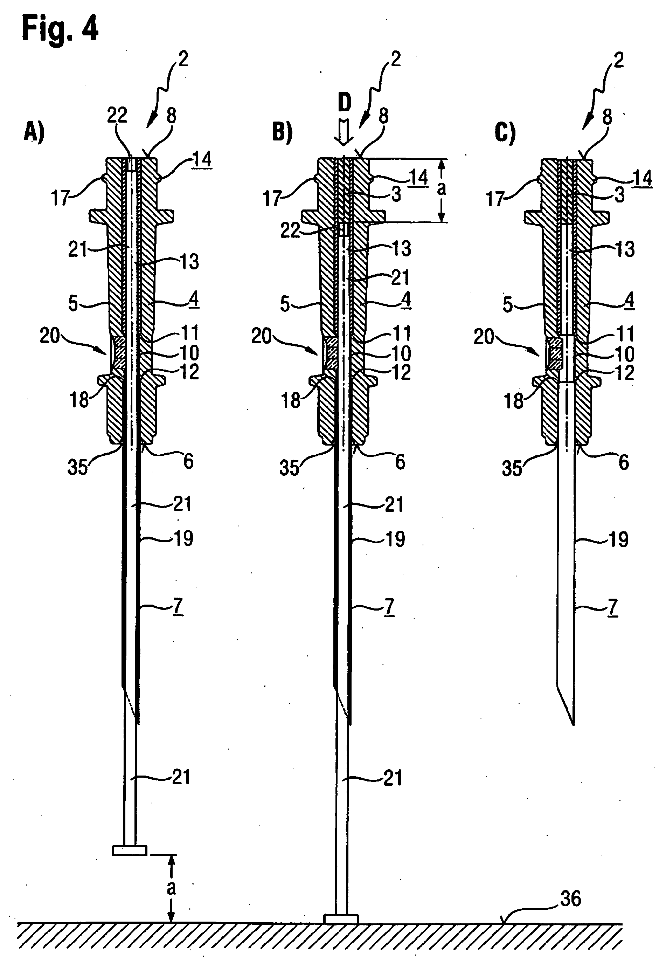

Figur 4 zeigt mit den schematischen Ansichten A), B) und C) das Bestücken einer Implantationsvorrichtung 2 mit einem Implantat. Im Unterschied zur Figur 3 sind bei diesem Bestücken bereits die Kanülen 7 mit ihrem distalen Ende 18 in der proximalen Öffnung 35 des Kanülenhalters 4 angeordnet, wobei das distale Ende 18 an den Absatz 12 des Kanülenhalters 4 anstößt. Um die Länge des einzubringenden Implantatmaterials zu definieren, ist ein gegenüber dem in Figur 3 dargestellten Kolbenstab um die Länge der Kanüle 7 längerer Kolbenstab 21 vorgesehen. Komponenten in den Figuren 4 A), B) und C), welche die gleichen Funktionen aufweisen wie in Figur 3, werden mit gleichen Bezugszeichen gekennzeichnet und nicht extra erörtert. Somit wird mit Figur 4 lediglich demonstriert, dass es auch möglich ist, bei bereits eingesetzter Kanüle 7 die Implantatkammer 13 mit einer vorbestimmten Menge an Wirkstoff-Polymer-Mischung zu bestücken. Anschließend kann wiederum eine Sterilisation erfolgen und ein mit einem Implantat 3 bestückter Kanülenhalter 4 mit angebrachter Kanüle 7 ausgeliefert werden.

Alternativ zur Bestückung des Kanülenhalters von seinem distalen Ende aus kann der Kanülenhalter auch von seinem proximalen Ende aus mit eine Implantat bestückt werden, insbesondere, wenn der Kanülenhalter erst nach dem Bestücken mit der Kanüle versehen wird.

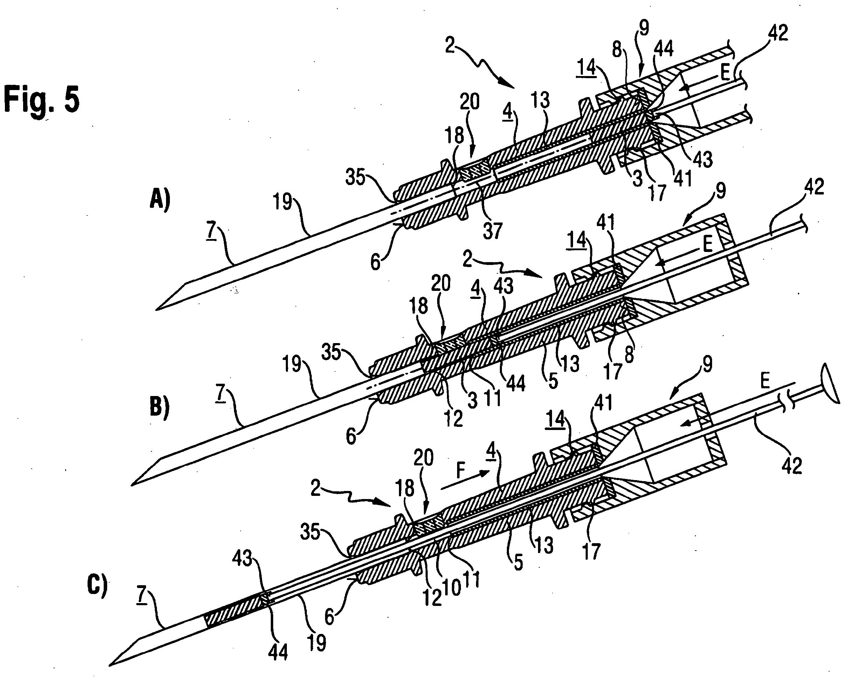

Figur 5 zeigt mit den schematischen Ansichten A), B) und C) eine Implantationsvorrichtung 2 beim Applizieren des Implantats 3. Dazu wird, wie Figur 5 A) zeigt, ein Applikator 9 auf das distale Ende 8 mit Hilfe der Koppeleinrichtung 14 aufgesetzt, wobei das Mundstück des Applikators eine scheibenförmige Dichtung 41 aufweist, die das distale Ende 8 des Kanülenhalters 4 mit dem Applikator 9 mediendicht verbindet. Vorzugsweise weist das Mundstück des Applikators 9 ein Luer-Lock-Innengewinde auf, das in ein Luer-Lock-Außengewinde 17 des Kanülenhalters 4 eingreift. Ein zentraler Applikatorstab 42 wird in eine koaxiale Position zu der Kanülenhalterachse 37 gebracht und kontaktiert mit einem Dichtkolben 43, der eine medizinisch inerte Beschichtung 44 aufweist, das distale Ende des Implantats 3.

In Figur 5 B) wird in Pfeilrichtung E ein Druck auf das Implantat 3 ausgeübt, so dass das Implantat 3 aus der Implantatkammer 13 herausgedrückt und an dem Sichtfenster 20 vorbeigeführt wird, womit eine Kontrolle durch das applizierende Personal möglich ist.

In Figur 5 C) wurde in Pfeilrichtung E mit Hilfe des Applikatorstabs 42 das Implantat 3 in die Injektionsnadel 19 verbracht und kann, wenn die Injektionsnadel 19 bereits subkutan angeordnet ist, schließlich subkutan aus der Injektionsnadel 19 herausgedrückt und positioniert werden. Dazu ist eine gleichzeitige Bewegung der Injektionsnadel 19 in distaler Richtung vorteilhaft, um das Implantat 3 subkutan zu positionieren.

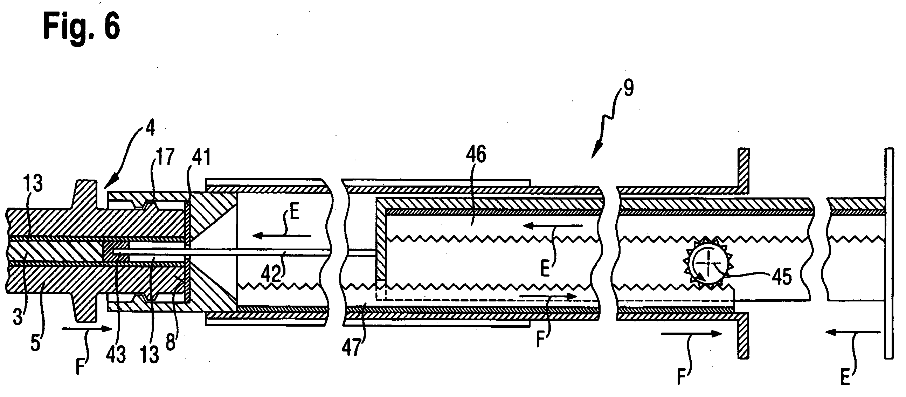

Figur 6 zeigt eine Prinzipskizze eines neuen Applikators 9, der ein Getrieberad 45 aufweist. Dieses koppelt zwei Zahnstangen 46 und 47 miteinander und bewirkt, dass beim Eindrücken des Implantats 3 in Richtung E synchron ein Zurückziehen der Injektionsnadel in Richtung F erfolgt. Bevor jedoch diese synchrone Bewegung erfolgt, wird das Implantat 3 über die in Figur 5 C) gezeigte Position hinaus an das proximale Ende der Kanüle 7 gebracht.

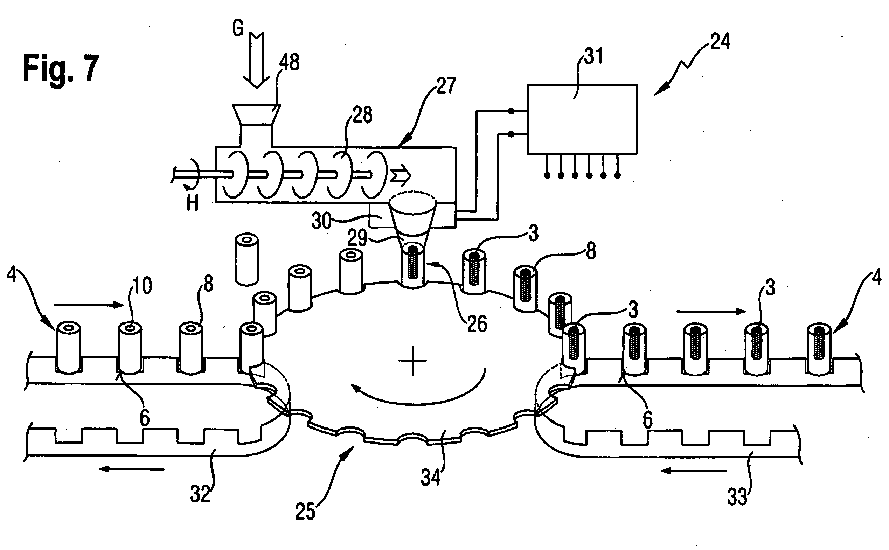

Figur 7 zeigt eine teilweise perspektivische Prinzipskizze einer Bestückungsvorrichtung 24 zum Bestücken von Kanülenhaltern 4 mit einem Implantat 3. Dazu weist die Bestückungsvorrichtung 24 eine Zufuhrvorrichtung 25 mit in dieser Ausführungsform der Erfindung zwei Transportbändern 32 und 33 sowie einem Drehteller 34 und einem Extruder 27 mit einer Mischeinrichtung 28 auf. Die gesamte Bestückungsvorrichtung 24 wird von einem Steuer- und Überwachungsgerät 31 geregelt. Ein Polymergranulat und der Wirkstoff werden in Pfeilrichtung G in die Öffnung 48 des Extruders 27 eingebracht und über einen Schneckenvortrieb durch Drehen in Pfeilrichtung H in eine Mischeinrichtung 28 verbracht, wobei gleichzeitig ein Druck aufgebaut wird, der das Gemisch aus Wirkstoff und Polymergranulat zu einer Extrusionsdüse 29 presst. Ein Heizer 30 erwärmt die Extrusionsdüse 29 auf eine Erweichungstemperatur des Polymergranulats, so dass dieses flüssig oder zumindest zähflüssig bzw. unter Druck fließfähig wird.

Über ein Zuführtransportband 32 werden die Kanülenhalter 4 mit leerer, unbefüllter Implantatkammer dem Drehteller 34 zugeführt, der sie taktweise unter die Extrusionsdüse 29 in eine Bestückungsposition 26 transportiert. Unter Öffnen und Schließen der Extrusionsdüse 29 wird in der Bestückungsposition 26 eine vorher mittels eines Kolbenstabs eingestellte Menge an Implantatmaterial in Implantatkammern der Kanülenhalter 4 eingefüllt.

Die mit Implantatmaterial bestückten bzw. befüllten Kanülenhalter 4 werden an ihren distalen Enden 8 mit in den Figuren 1B) und 2B) gezeigten Verschlusskappen versehen und auf ihren proximalen Enden 6 mit entsprechenden Stopfen abgedichtet und einem Abnahmetransportband 33 zugeführt. Die Transportbänder 32 und 33 können als Endlosbänder ausgeführt sein und werden von einem hier nicht gezeigten Automaten bestückt bzw. entleert. Auf dem Entnahmetransportband 33 können Sterilisationspositionen vorgesehen werden, bevor die befüllten bzw. bestückten Kanülenhalter 4 von dem Abnahmetransportband 33 entnommen und steril verpackt versandt werden.

### Bezugszeichenliste

- 1: Implantationsvorrichtung (erste Ausführungsform)
- 2: Implantationsvorrichtung (zweite Ausführungsform)
- 3: Implantat
- 4: Kanülenhalter
- 5: Gehäuse des Kanülenhalters
- 6: proximales Ende des Kanülenhalters
- 7: Kanüle
- 8: distales Ende des Kanülenhalter
- 9: Applikator
- 10: Implantatkanal
- 11: Absatz im Kanal
- 12: Absatz im Kanal
- 13: Implantatkammer
- 14: Koppeleinrichtung
- 15: Verschlusskappe
- 16: Stopfen
- 17: Luer-Lock-Außengewinde
- 18: distales Ende der Kanüle
- 19: Injektionsnadel
- 20: Sichtfenster
- 21: Kolbenstab
- 22: Dichtkolben
- 23: Beschichtung des Dichtkolbens
- 24: Bestückungsvorrichtung
- 25: Zufuhrvorrichtung
- 26: Bestückungsposition
- 27: Extruder
- 28: Mischeinrichtung
- 29: Extrusionsdüse
- 30: Heizer
- 31: Steuer- und Überwachungsgerät
- 32: Transportband
- 33: Transportband
- 34: Drehteller
- 35: proximale Öffnung des Kanülenhalters
- 36: Anschlag
- 37: Kanülenhalterachse
- 38: Rohr
- 39: Dichtungsscheibe
- 40: distales Ende des Kolbenstabs
- 41: Dichtung
- 42: Applikatorstab
- 43: Dichtkolben
- 44: Beschichtung
- 45: Getrieberad
- 46: Zahnstange
- 47: Zahnstange
- 48: Öffnung
- 49: proximales Ende des Kolbenstabs

- a: Abstand
- D: Pfeilrichtung
- E: Pfeilrichtung
- F: Pfeilrichtung
- G: Pfeilrichtung
- H: Pfeilrichtung
- l: Länge des Implantatstäbchens

## Patentansprüche

1. Kanülenvorrichtung (4) mit:
einem proximalen Ende (6);
einem distalen Ende (8), das zur Verbindung mit einem Applikator (9) vorgesehen ist;
einem Implantatkanal (10), der sich von dem distalen Ende (8) zu dem proximalen Ende (6) der Kanülenvorrichtung (4) erstreckt;
charakterisiert durch
eine schlauch- oder rohrförmige Auskleidung (38) von zumindest einem Abschnitt des Implantatkanals (10).

2. Kanülenvorrichtung nach Anspruch 1, ferner mit einem Implantat (3) in dem Implantatkanal (10), wobei das Implantat (3) durch Verfestigen eines in fließfähigem Zustand in den Implantatkanal (10) eingebrachten Materials in dem Implantatkanal (10) gebildet ist.

3. Kanülenvorrichtung (4) nach einem der Ansprüche 1 oder 2, ferner mit:
einer Bohrung in der Kanülenvorrichtung (4), die einen Innendurchmesser aufweist, der größer ist als ein Innendurchmesser des Implantatkanals (10),
wobei die Auskleidung (38) durch ein in die Bohrung eingesetztes Rohr gebildet wird, dessen Innenraum zumindest einen Abschnitt des Implantatkanals (10) bildet.

4. Kanülenvorrichtung (4) nach dem vorangehenden Anspruch, bei der die Bohrung von einem Ende der Kanülenvorrichtung (4) ausgeht, und bei der die Bohrung und das Rohr jeweils eine geringere Länge aufweisen als der Implantatkanal (10).

5. Kanülenvorrichtung (4) nach einem der vorangehenden Ansprüche, bei der der Implantatkanal (10) im Bereich des Implantats (3) einen geringeren Innendurchmesser aufweist als an anderen Stellen.

6. Kanülenvorrichtung (4) nach einem der vorangehenden Ansprüche, ferner mit einer Kanüle (7), wobei der Implantatkanal (10) zumindest im Bereich des Implantats (3) einen geringeren Innendurchmesser aufweist als die Kanüle (7).

7. Kanülenvorrichtung (4) nach einem der vorangehenden Ansprüche, wobei die Kanülenvorrichtung (4) ein transparentes Material oder ein Sichtfenster (20) aus transparentem Material aufweist.

8. Kanülenvorrichtung (4) nach Anspruch 2 oder nach einem der davon abhängigen vorangehenden Ansprüche, wobei das Implantat als Wirkstoff Goserelin und/oder Leuprorelin umfasst.

9. System, umfassend eine Kanülenvorrichtung (4) nach einem der vorangehenden Ansprüche und eine Bestückungsvorrichtung (24) zum Bestücken des Implantatkanals (10) der Kanülenvorrichtung (4) mit einem Implantat (3), wobei die Bestückungsvorrichtung (24) aufweist:
eine Extrusionsdüse (29) zum Extrudieren eines fließfähigen Implantatmaterials;
eine Dichtfläche an der Extrusionsdüse;
eine Haltevorrichtung (25) zum Halten einer Kanülenvorrichtung an der Extrusionsdüse (29) derart, dass die Kanülenvorrichtung an der Dichtfläche anliegt; und
eine Materialzuführeinrichtung (27) zum Zuführen des fließfähigen Implantatmaterials durch die Extrusionsdüse (29) in den Implantatkanal (10).

10. System nach Anspruch 9, wobei die Bestückungsvorrichtung (24) ferner einen Anschlag für einen Kolbenstab (21) aufweist, der in einem Implantatkanal (10) einer von der Halteeinrichtung (25) an der Extrusionsdüse (29) gehaltenen Kanülenvorrichtung (4) angeordnet und entlang des Implantatkanals (10) bewegbar ist.

11. Verfahren zum Bestücken einer Kanülenvorrichtung (4) mit einem Implantat (3), mit folgenden Schritten:
Halten der Kanülenvorrichtung (4) an einer Extrusionsdüse (29) so, dass die Kanülenvorrichtung (4) an einer Dichtfläche der Extrusionsdüse (29) anliegt;
Zuführen eines fließfähigen Implantatmaterials durch die Extrusionsdüse (29) in einen Implantatkanal (10) der Kanülenvorrichtung (4);
Verfestigen des Implantatmaterials in dem Implantatkanal (10), insbesondere durch Abkühlen; und
wobei die Kanülenvorrichtung (4) eine Bohrung aufweist, deren Innendurchmesser größer ist als ein Innendurchmesser des Implantatkanals (10), dadurch charakterisiert, dass vor dem Zuführen des fließfähigen Implantatmaterials ein Rohr (38) in die Bohrung eingesetzt wird, dessen Innenraum einen Teil des Implantatkanals (10) bildet.

12. Verfahren nach Anspruch 11, wobei vor dem Zuführen des fließfähigen Implantatmaterials ein Kolbenstab (21) in den Implantatkanal eingeführt wird, und wobei der Kolbenstab (21) beim Zuführen des Implantatmaterials zumindest teilweise aus dem Implantatkanal (10) verdrängt wird.

13. Verfahren nach Anspruch 11 oder 12, wobei das fließfähige Implantatmaterial und das Implantat als Wirkstoff Goserelin und/oder Leuprorelin umfassen.

## Claims

1. Cannula device (4) having:
a proximal end (6);
a distal end (8), which is provided for connection to an applicator (9);
an implant channel (10), which extends from the distal end (8) to the proximal end (6) of the cannula device (4);
**characterized by**
a hose- or tube-like lining (38) of at least a portion of the implant channel (10).

2. Cannula device (4) according to claim 1, further having an implant (3) in the implant channel (10), wherein the implant (3) is formed by solidification in the implant channel (10) of a material that is introduced into the implant channel (10) in a flowable state.

3. Cannula device (4) according to any one of claims 1 or 2, further having:
a bore in the cannula device (4), which bore has an inside diameter which is larger than the inside diameter of the implant channel (10),
wherein the lining (38) is formed by a tube inserted into the bore, the inside space of which tube forms at least a portion of the implant channel (10).

4. Cannula device (4) according to the preceding claim, in which the bore starts at one end of the cannula device (4), and in which the bore and the tube each have a shorter length than the implant channel (10).

5. Cannula device (4) according to any one of the preceding claims, in which the implant channel (10) has a smaller inside diameter in the region of the implant (3) than in other places.

6. Cannula device (4) according to any one of the preceding claims, further having a cannula (7), wherein the implant channel (10) has a smaller inside diameter than the cannula (7) at least in the region of the implant (3).

7. Cannula device (4) according to any one of the preceding claims, wherein the cannula device (4) has a transparent material or a viewing window (20) of transparent material.

8. Cannula device (4) according to claim 2 or according to any one of the preceding claims dependent thereof, wherein the implant comprises goserelin and/or leuprorelin as active ingredient.

9. System, comprising a cannula device (4) according to any one of the preceeding claims and a
loading device (24) for loading the implant channel (10) of the cannula device (4) with an implant (3), wherein the loading device (24) comprises:
an extrusion die (29) for extruding a flowable implant material;
a sealing surface on the extrusion die;
a holding device (25) for holding a cannula device at the extrusion die (29) in such a manner that the cannula device is in contact with the sealing surface; and
a material feed device (27) for feeding the flowable implant material through the extrusion die (29) into the implant channel (10).

10. System according to claim 9, wherein the loading device (24) further has a stop for a piston rod (21), which is arranged in an implant channel (10) of a cannula device (4) held at the extrusion die (29) by the holding device (25) and is movable along the implant channel (10).

11. Method for loading a cannula device (4) with an implant (3), comprising the following steps:
holding the cannula device (4) at an extrusion die (29) in such a manner that the cannula device (4) is in contact with a sealing surface of the extrusion die (29);
feeding a flowable implant material through the extrusion die (29) into an implant channel (10) of the cannula device (4) ;
solidifying the implant material in the implant channel (10), especially by cooling; and
wherein the cannula device (4) has a bore, the inside diameter of which is larger than an inside diameter of the implant channel (10), **characterized in that**, before the flowable implant material is fed in, a tube (38) is inserted into the bore, the inside space of which tube forms part of the implant channel (10).

12. Method according to claim 11, wherein a piston rod (21) is introduced into the implant channel before the flowable implant material is fed in, and wherein the piston rod (21) is displaced at least partly out of the implant channel (10) when the implant material is fed in.

13. Method according to claim 11 or 12, wherein the flowable implant material and the implant comprise goserelin and/or leuprorelin as active ingredient.

## Revendications

1. Dispositif de canule (4) comprenant:
une extrémité proximale (6);
une extrémité distale (8), qui est prévue pour être reliée à un applicateur (9);
un canal d'implant (10), qui s'étend de l'extrémité distale (8) vers l'extrémité proximale (6) du dispositif de canule (4);
**caractérisé par**
un habillage (38) en forme de tuyau flexible ou de tube d'au moins un tronçon du canal d'implant (10).

2. Dispositif de canule selon la revendication 1, comprenant en outre un implant (3) dans le canal d'implant (10), l'implant (3) étant formé dans le canal d'implant (10) par la solidification d'un matériau introduit dans un état où il peut s'écouler dans le canal d'implant (10).

3. Dispositif de canule (4) selon l'une quelconque des revendications 1 ou 2, comprenant en outre:
un alésage dans le dispositif de canule (4), qui présente un diamètre intérieur qui est plus grand qu'un diamètre intérieur du canal d'implant (10),
l'habillage (38) étant formé par un tube inséré dans l'alésage, dont l'espace intérieur forme au moins un tronçon du canal d'implant (10).

4. Dispositif de canule (4) selon la revendication précédente, dans le cadre duquel l'alésage part d'une extrémité du dispositif de canule (4), et dans le cadre duquel l'alésage et le tube présentent respectivement une longueur une longueur inférieure à celle du canal d'implant (10) .

5. Dispositif de canule (4) selon l'une quelconque des revendications précédentes, dans le cadre duquel le canal d'implant (10) présente dans la zone de l'implant (3) un diamètre intérieur inférieur à celui d'autres emplacements.

6. Dispositif de canule (4) selon l'une quelconque des revendications précédentes, comprenant en outre une canule (7), le canal d'implant (10) présentant au moins dans la zone de l'implant (3) un diamètre intérieur inférieur à celui de la canule (7).

7. Dispositif de canule (4) selon l'une quelconque des revendications précédentes, le dispositif de canule (4) présentant un matériau transparent ou une fenêtre de contrôle (20) composée d'un matériau transparent.

8. Dispositif de canule (4) selon la revendication 2 ou selon l'une quelconque des revendications précédentes dépendantes de celle-ci, l'implant comprenant en tant que principe actif de la goseréline et/ou de la leuproréline.

9. Système, comprenant un dispositif de canule (4) selon l'une quelconque des revendications précédentes et comprenant un dispositif d'équipement (24) servant à équiper le canal d'implant (10) du dispositif de canule (4) avec un implant (3), le dispositif d'équipement (24) présentant:
une tuyère d'extrusion (29) servant à extruder un matériau d'implant pouvant s'écouler;
une face étanche au niveau de la tuyère d'extrusion;
un dispositif de maintien (25) servant à maintenir un dispositif de canule au niveau de la tuyère d'extrusion (29) de telle manière que le dispositif de canule repose au niveau de la face étanche; et
un système d'amenée de matériau (27) servant à amener le matériau d'implant pouvant s'écouler en passant par la tuyère d'extrusion (29) dans le canal d'implant (10).

10. Système selon la revendication 9, le dispositif d'équipement (24) présentant en outre une butée pour une tige de piston (21), laquelle est disposée dans un canal d'implant (10) d'un dispositif de canule (4) maintenu au niveau de la tuyère d'extrusion (29) par le système de maintien (25) et qui peut être déplacée le long du canal d'implant (10).

11. Procédé servant à équiper un dispositif de canule (4) avec un implant (3), comprenant les étapes suivantes consistant à:
maintenir le dispositif de canule (4) au niveau de la tuyère d'extrusion (29) de sorte que le dispositif de canule (4) repose au niveau d'une face étanche de la tuyère d'extrusion (29);
amener un matériau d'implant pouvant s'écouler en passant par la tuyère d'extrusion (29) dans un canal d'implant (10) du dispositif de canule (4);
solidifier le matériau d'implant dans le canal d'implant (10), en particulier par refroidissement; et
le dispositif de canule (4) présentant un alésage, dont le diamètre intérieur est plus grand qu'un diamètre intérieur du canal d'implant (10), **caractérisé en ce qu'**un tube (38) est inséré dans l'alésage avant l'amenée du matériau d'implant pouvant s'écouler, dont l'espace intérieur forme une partie du canal d'implant (10).

12. Procédé selon la revendication 11, une tige de piston (21) étant introduite dans le canal d'implant avant l'amenée du matériau d'implant pouvant s'écouler, et la tige de piston (21) étant refoulée, lors de l'amenée du matériau d'implant, au moins en partie hors du canal d'implant (10).

13. Procédé selon la revendication 11 ou 12, le matériau d'implant pouvant s'écouler et l'implant comprenant en tant que principe actif de la goseréline et/ou de la leuproréline.
